# EUROPÄISCHE PATENTSCHRIFT

(11) **EP 1 516 638 B1**
(45) Veröffentlichungstag und Bekanntmachung des Hinweises auf die Patenterteilung: **13.01.2010**
(21) Anmeldenummer: 04020575.9
(22) Anmeldetag: 31.08.2004
(51) Int. Cl.: A61M 5/315, A61M 5/24

(54) **Vorrichtung für eine dosierte Abgabe eines injizierbaren Produkts**
Device for a dosed release of an injectable product
Appareil pour la libération dosée d'un produit injectable

(30) Priorität: 19.09.2003 DE 10343548
(43) Veröffentlichungstag der Anmeldung: 23.03.2005
(73) Patentinhaber: TecPharma Licensing AG, 3401 Burgdorf (CH)
(72) Erfinder: Jost, Stefan, 3203 Mühleberg (CH)
(74) Vertreter: Schwabe - Sandmair - Marx

(56) Entgegenhaltungen:
- EP-A1- 0 778 034
- WO-A1-2004/002557
- WO-A2-94/22507
- US-B1- 6 193 698

## Beschreibung

Die Erfindung bezieht sich auf eine Vorrichtung zur dosierten Abgabe eines bevorzugt injizierbaren Produktes, wie z.B. Insulin oder Hormonpräparate.

Es sind Vorrichtungen bekannt, bei welchen ein injizierbares Produkt abgegeben wird und eine Rücksetzung der Dosiereinrichtung der Vorrichtung entweder sehr umständlich oder überhaupt nicht möglich ist.

In der EP 0 614 386 B1 ist eine Vorrichtung beschrieben, bei welcher eine Zahnstange, die auf einen in einem Behältnis für das dosierbare Produkt angeordneten Kolben wirkt, nur über einen Teil ihres Umfanges mit Zähnen versehen ist. Die Zähne der Zahnstange wirken mit Gegenzähnen derart zusammen, dass ein Verschieben der Zahnstange zum Zwecke der Produktausschüttung in diskreten Schritten möglich ist, aber ein einfaches Zurückschieben der Zahnstange verhindert wird. Für ein Zurücksetzen der Zahnstange ist diese so ausgebildet, dass die Zähne und die Gegenzähne durch Drehung der Zahnstange um 90° außer Eingriff gebracht werden und die Zahnstange anschließend manuell zurückgezogen und in eine Ausgangsstellung für eine erneute, dosierte Ausschüttung gebracht werden kann. Das Zurücksetzen des Abtriebsglieds erfordert von einem Verwender die koordinierte Ausführung mehrerer Bewegungen. Dies kann jedoch für einen Benutzer, welcher möglicherweise mit der Handhabung einer etwas komplexeren Mechanik nicht vertraut ist, zu Problemen führen und damit den Anwendungsbereich der Vorrichtung einschränken.

Aus der DE 10 046 279 A1 der Anmelderin ist eine Vorrichtung zur dosierten Verabreichung eines injizierbaren Produktes bekannt, wobei eine in einem gespannten Zustand gesicherte Rücksetz-Spiralfeder vorgesehen ist, welche durch Entsicherung mit einer Antriebseinheit gekoppelt wird und eine Rücksetzbewegung der Antriebseinheit in Richtung auf ihre Dosierausgangsstellung bewirken kann.

WO 94/22507 offenbart eine Injektionsvorrichtung gemäß dem Oberbegriff des Anspruchs 1, welche einen Mechanismus besitzt, der es der Kolbenstange erlaubt, sich in die Ausgangsposition zurück zu verschieben. Dieser Mechanismus erlaubt oder verhindert, dass die Kolbenstange Drehbewegungen ausführen kann.

US 6,193,698 B1 und EP 0 778 034 A1 offenbaren eine Injektionsvorrichtung, in der die Kolbenstange von einer Feder angetrieben in ihre Ausgangsposition zurück verschoben werden kann.

WO 2004/002557 A1 stellt Stand der Technik gemäß Artikel 54(3) EPÜ dar und offenbart ein Verabreichungsgerät mit rücksetzbarer Betätigungssperre, welches eine Verschiebevorrichtung mit Dosiergliedern besitzt und eine Rückstellfeder, mit der die Kolbenstange in ihre Ausgangsposition zurück verschoben werden kann.

Es ist eine Aufgabe der Erfindung eine Vorrichtung zur dosierten Abgabe eines injizierbaren Produktes vorzuschlagen, bei welcher eine automatische Rückführung einer Dosiervorrichtung möglich ist, bei der sich die Anzahl der möglichen Bedienungsfehler reduzieren soll. Insbesondere soll eine solche Vorrichtung vorgeschlagen werden, bei welcher ein Rückstellelement bevorzugt bei einer Vielzahl von Rückstellvorgängen eingesetzt werden kann und welches möglichst platzsparend in die Vorrichtung integriert werden kann.

Diese Aufgabe wird durch die Vorrichtung nach Anspruch 1 gelöst. Vorteilhafte Ausführungsformen ergeben sich aus den Unteransprüchen.

Die erfindungsgemäße Vorrichtung zur dosierten Abgabe eines injizierbaren Produkts, wie z.B. Insulin oder Hormonpräparate, weist ein Gehäuseelement oder einen Mechanikhalter auf, welcher Teile der Dosiermechanik oder die vollständige Dosiermechanik aufnehmen kann. Eine Dosiervorrichtung, wie z.B. eine Gewindestange, ist relativ zu dem Gehäuseelement und bevorzugt in diesem verschiebbar gelagert, wobei z.B. eine Ampulle, welche das zu dosierende injizierbare Produkt enthält, mit dem Gehäuseelement verbunden werden kann und durch eine Verschiebung der Dosiervorrichtung relativ zum Gehäuseelement eine bestimmte Menge der in der Ampulle enthaltene Substanz dosiert und nach Betätigung eines Auslöseknopfes abgegeben werden kann. Mit einer Einstellvorrichtung, welche z.B. relativ zur Dosiervorrichtung und/oder zum Gehäuseelement drehbar ist, kann die Position der Dosiervorrichtung eingestellt oder verändert werden. Erfindungsgemäß ist ein Rückstellelement in der oder an der Vorrichtung vorgesehen, welches eine Rückstellkraft erzeugen kann, durch welche die Dosiervorrichtung nach einem oder mehreren Dosiervorgängen automatisch wieder in die Ausgangsstellung zurückgebracht werden kann, um beispielsweise eine teilweise oder vollständig geleerte Ampulle auszuwechseln und durch eine neue gefüllte Ampulle zu ersetzen. Das Rückstellelement ist bevorzugt so ausgebildet und angeordnet, dass die zum Zurückstellen der Dosiervorrichtung erforderliche Kraft während eines Einstell- und/oder Abgabevorgangs der Vorrichtung aufgebaut wird und die so aufgebaute Kraft z.B. nach einem Entsichern des Rückstellelements auf die Dosiervorrichtung übertragen werden kann, um diese in eine Ausgangsstellung zu bringen.

Unter einer "Dosiervorrichtung" soll in dieser Anmeldung eine Vorrichtung verstanden werden, mit welcher die Menge einer z.B. aus einer Ampulle abzugebenden Substanz eingestellt werden kann. Je nach Bauart ist es auch möglich, dass die Dosiervorrichtung auch zum Abgeben der Substanz z.B. durch Verschieben der Dosiervorrichtung verwendet wird.

Bevorzugt ist das Rückstellelement ein Federelement und besonders bevorzugt eine Schraubenfeder oder eine Biegefeder, welche z.B. durch eine Bewegung der Dosiervorrichtung in Abgaberichtung komprimiert werden kann und so die zum Zurückstellen der Dosiervorrichtung erforderliche Kraft aufbaut. Insbesondere ist die Verwendung einer Schraubenfeder vorteilhaft, da diese ohne großen Platzbedarf in eine Abgabevorrichtung integriert werden kann, indem diese Schraubenfeder z.B. um eine als Dosiervorrichtung verwendete Gewindestange herum angeordnet ist. Somit müssen keine weiteren separaten Elemente an der Abgabevorrichtung angebracht werden, was den Aufbau der Abgabevorrichtung vereinfacht. Vorteilhaft ist das Rückstellelement oder das Federelement koaxial zu der Dosiervorrichtung, dem Gehäuseelement und/oder bevorzugt zumindest zum Teil innerhalb des Gehäuseelements angeordnet und kann eine Kraft in einer axialen Richtung des Gehäuseelements und/oder in Bewegungsrichtung der Dosiervorrichtung erzeugen, indem es z.B. zwischen der Dosiervorrichtung und dem Gehäuseelement selbst oder zwischen der Dosiervorrichtung und einem in das Gehäuseelement einbringbaren Element, wie z.B. einem später beschriebenen Verschlussteil angeordnet ist.

Bevorzugt ist eine Sicherung vorgesehen, welche ein Rückstellen der Dosiervorrichtung durch das Rückstelleelement verhindert. Diese Sicherung kann z.B. ein Schalter oder eine nachfolgend beschriebene Verdrehsicherung sein.

Vorteilhaft ist ein Gewindeeingriff zwischen der Einstellvorrichtung und der Dosiervorrichtung vorgesehen, wobei besonders bevorzugt die Einstellvorrichtung koaxial zur Dosiervorrichtung angeordnet ist. Beispielsweise kann die Einstellvorrichtung ein Innengewinde haben, welches mit der Dosiervorrichtung, die z.B. durch eine Gewindestange mit Außengewinde gebildet wird, in Eingriff bebracht werden kann. Ebenso ist es möglich, dass die Einstellvorrichtung ein Außengewinde aufweist, welches mit einem Innengewinde der Dosiervorrichtung in Eingriff gebracht werden kann.

Weist die Dosiervorrichtung ein Gewinde auf, welches z.B. in ein korrespondierendes Gewinde der Einstellvorrichtung eingreift, so ist es vorteilhaft, das Gewinde so auszubilden, dass es nicht selbsthemmend ist, d.h. die Gewindesteigung sollte so gewählt werden, dass die durch das Rückstellelement an die Dosiervorrichtung angelegte Kraft ausreicht, um die Dosiervorrichtung in Richtung auf eine Ausgangsposition zu bewegen.

Erfindungsgemäß kann die Dosiervorrichtung relativ zu dem Gehäuseelement z.B. während eines Dosiervorganges und/oder eines Abgabevorganges verdrehgesichert werden, beispielsweise indem die Dosiervorrichtung keinen rotationssymmetrischen Querschnitt aufweist, so dass eine diesem Querschnitt entsprechende Durchgangsöffnung, welche in einem relativ zu dem Gehäuseelement verdrehsicher gelagerten Element angeordnet ist, die Dosiervorrichtung verdrehsicher in dem Gehäuseelement halten kann. Dabei kann die Dosiervorrichtung z.B. noch durch diese Verdrehsicherung frei hindurch bewegt werden. Wird das zur Verdrehsicherung der Dosiervorrichtung dienende Element relativ zum Gehäuseelement freigegeben, so kann auch die Dosiervorrichtung relativ zum Gehäuseelement bewegt und insbesondere gedreht werden. Im verdrehgesicherten Zustand der Dosiervorrichtung kann somit beispielsweise über ein Drehen der über ein Gewinde mit der Dosiervorrichtung gekoppelten Einstellvorrichtung die Position der Dosiervorrichtung verändert werden, um eine Dosis der z.B. aus einer Ampulle abzugebenden Substanz einzustellen. Dabei ist vorteilhaft die Einstellvorrichtung relativ zum Gehäuseelement drehbar.

Erfindungsgemäß kann die Einstellvorrichtung relativ zum Gehäuseelement verdrehgesichert werden und bevorzugt kann die Dosiervorrichtung im verdrehgesicherten Zustand der Einstellvorrichtung relativ zum Gehäuseelement freibeweglich, insbesondere drehbar sein, wodurch es möglich ist, dass das erfindungsgemäß vorgesehene Rückstellelement die Dosiervorrichtung z.B. nach einer Entsicherung des Rückstellelements in eine Ausgangslage zurückbringt oder zurückschiebt. Dabei kann die Dosiervorrichtung z.B. über einen Gewindeeingriff mit der Einstellvorrichtung gekoppelt sein, wobei sich z.B. in diesem Fall die Dosiervorrichtung beim Zurücksetzen in die Ausgangsposition durch die Kraft des Rückstellelements zurückschraubt. Ebenso ist es möglich, dass der Gewindeeingriff oder allgemein jede Art der Kopplung der Einstellvorrichtung oder eines anderen Elements mit der Dosiervorrichtung gelöst wird, wodurch es möglich ist, dass die Dosiervorrichtung durch die von dem Rückstellelement erzeugte Kraft zurück in die Ausgangsstellung gebracht wird, in welcher die Einstellvorrichtung bevorzugt wieder mit der Dosiervorrichtung gekoppelt wird, z.B. durch eine Drehbewegung der Dosiervorrichtung und/oder der Rückstellvorrichtung, um wieder einen Gewindeeingriff zwischen diesen Vorrichtungen herzustellen.

Erfindungsgemäß sind die Verdrehsicherungen der Dosiervorrichtung und der Einstellvorrichtung so gekoppelt, dass in einem ersten Zustand die Einstellvorrichtung frei beweglich und die Dosiervorrichtung verdrehgesichert ist und in einem zweiten Zustand die Dosiervorrichtung frei beweglich und die Einstellvorrichtung verdrehgesichert ist. Unter dem Begriff "verdrehgesichert" soll im Sinne dieser Anmeldung eine Halterung oder Lagerung der betreffenden Vorrichtung verstanden werden, welche verhindert, dass diese Vorrichtung sich relativ zu einer anderen auf diese Vorrichtung einwirkende Vorrichtung verdreht, wodurch z.B. bei einem Gewindeeingriff eine Drehung einer mit der verdrehgesicherten Vorrichtung gekoppelten Vorrichtung bewirkt, dass die verdrehgesicherte Vorrichtung sich nicht mitdreht, sondern die Drehkraft in eine Vorschub- oder Rückschubbewegung umsetzt.

Gemäß einem weiteren Effekt bezieht sich die Erfindung auf ein System mit einer wie oben beschriebenen Vorrichtung und einer Koppelungsvorrichtung für eine Ampulle, in welche eine Ampulle eingesetzt oder aufgesteckt oder mit welcher allgemein eine Ampulle verbunden werden kann, wobei die Dosiervorrichtung z.B. bei oder nach einem Einstellvorgang in das Innere der Ampulle eingeschoben werden kann, um eine Verdrängung der in der Ampulle gespeicherten zur Abgabe einer bestimmten Substanz zu bewirken, wobei durch die Einschubtiefe der Dosiervorrichtung die Menge der aus der Ampulle abzugebenden Substanz bestimmt wird.

Vorteilhaft ist die Ampullen-Koppelungsvorrichtung so ausgestaltet, dass bei einer auf die Abgabevorrichtung aufgesteckten oder mit der Abgabevorrichtung verbundenen Ampulle die Dosiervorrichtung verdrehgesichert ist und z.B. durch ein Verdrehen der Einstellvorrichtung in die Ampulle hinein oder auf die Ampulle zu bewegt werden kann. Ist die Ampullenkopplung nicht mit einer Ampulle gekoppelt oder verbunden, wird vorteilhaft die Verdrehsicherung der Dosiervorrichtung gelöst, wodurch die Dosiervorrichtung z.B. durch das Rückstellelement in eine Ausgangsposition zurückgedreht werden kann.

Die Erfindung wird nachfolgend anhand eines Ausführungsbeispiels beschrieben werden.
Es zeigen:
- Figur 1: eine Ausführungsform der erfindungsgemäßen Vorrichtung in Explosionsansicht;
- Figur 2: die in Längsrichtung aufgeschnittene Vorrichtung von Figur 1;
- Figur 3: eine Querschnittansicht der in Figur 1 gezeigten Vorrichtung in zusammengesetzter Form in Ausgangsstellung;
- Figur 4: die in Figur 3 gezeigte Vorrichtung mit aufgesetzter Ampullenhülse in Ausgangsstellung;
- Figur 5: die in Figur 4 gezeigte Vorrichtung mit ausgeschobener Gewindestange; und

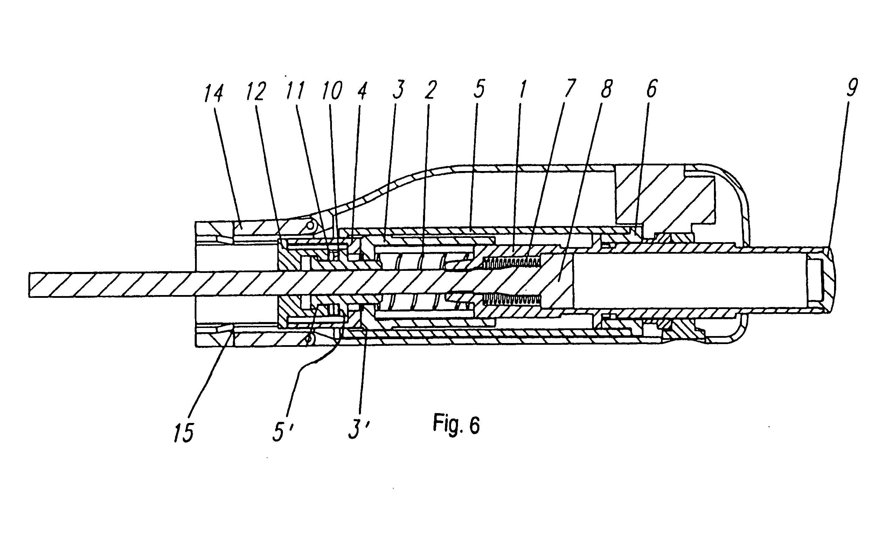

- Figur 6: die in Figur 5 gezeigte Vorrichtung nach Abnehmen der Ampulle;

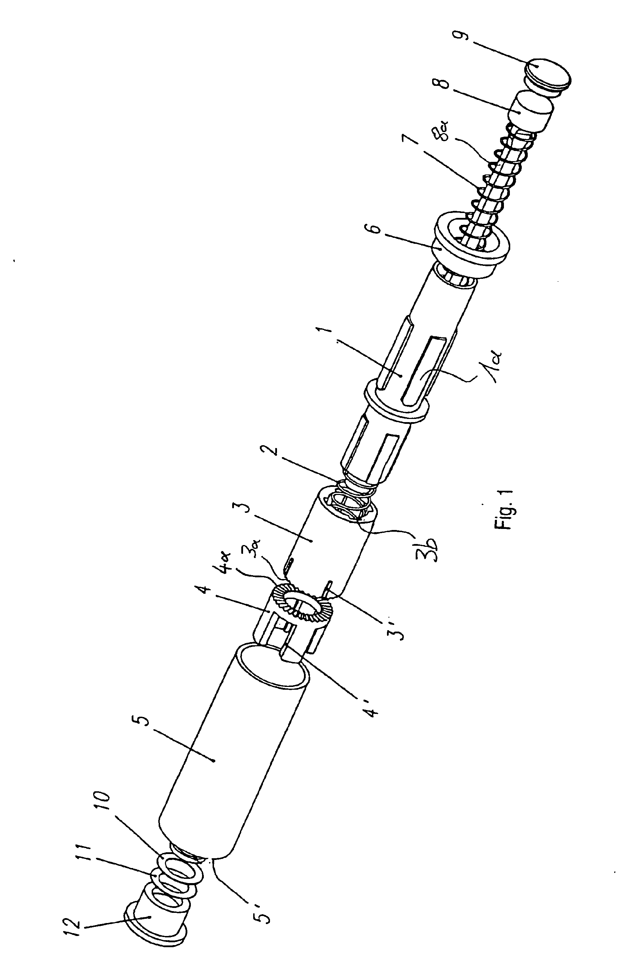

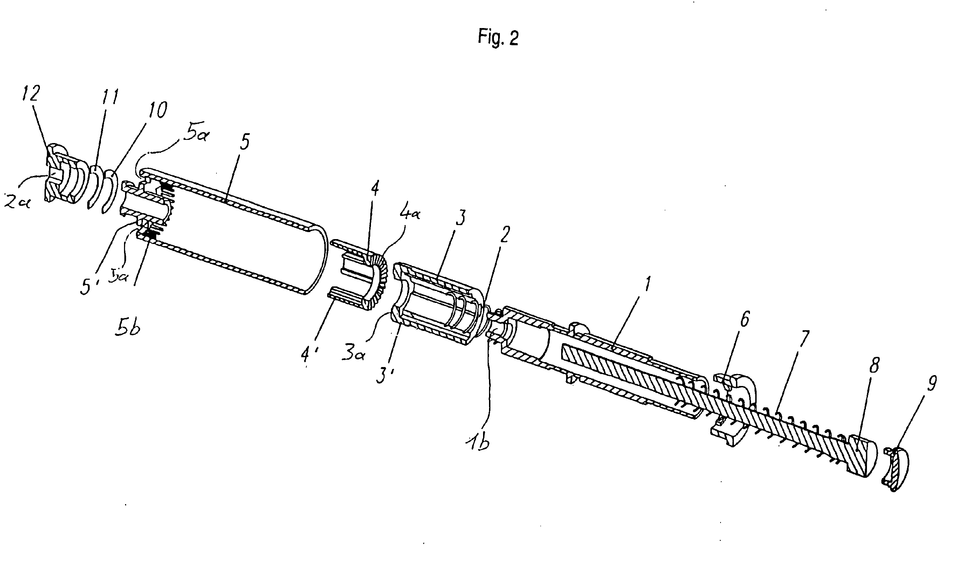

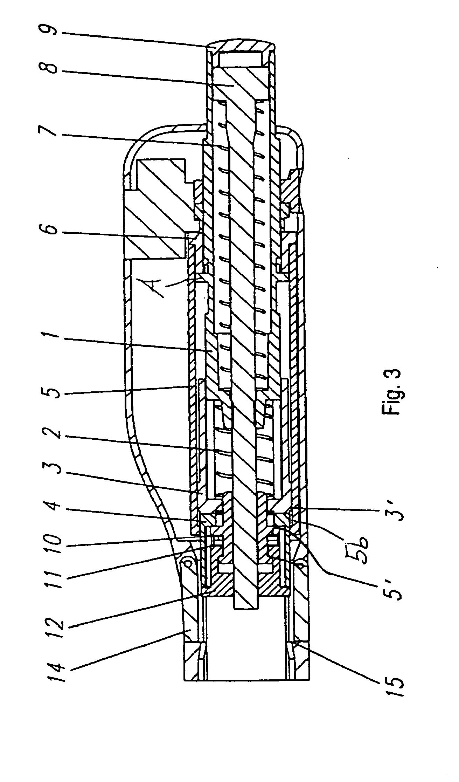

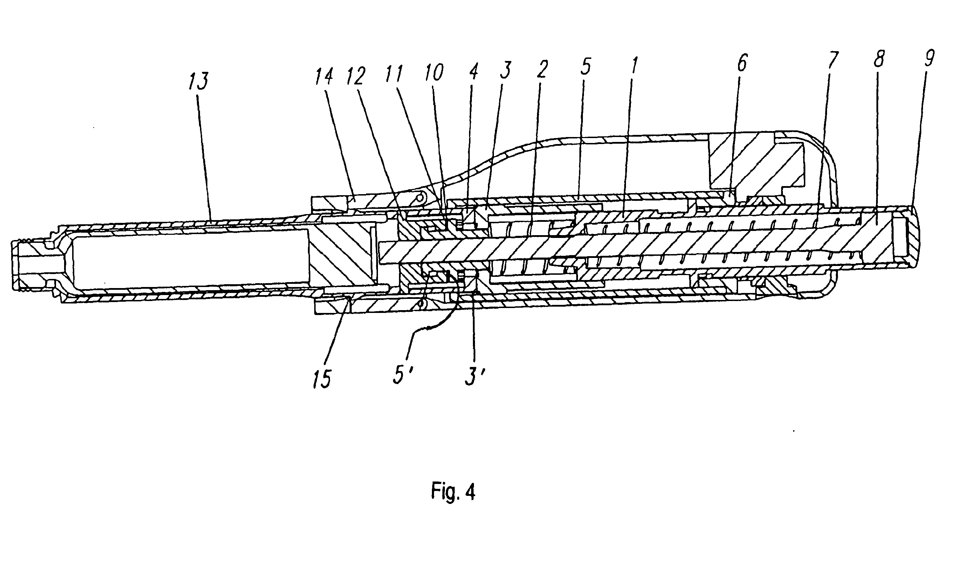

Figur 1 zeigt eine Ausführungsform der erfindungsgemäßen Vorrichtung mit einem als Gehäuseelement dienenden Mechanikhalter 5, welcher an seinem vorderen in Figur 1 links gezeigten Ende einen Anschlag 5' aufweist, auf welchem eine Schleifscheibe 10 angebracht ist. Gegenüberliegend zur Schleifscheibe 10 ist eine Schleifscheibe 11 angeordnet, welche auf einem Rotierring 12 befestigt ist. Im in Figur 3 gezeigten zusammengesetzten Zustand liegen die Schleifscheiben 10 und 11 nicht aufeinander auf und sind nicht durch einen zusätzlich von außen wirkenden Druck z.B. durch eine Ampulle, wie in Fig. 4 gezeigt, belastet, so dass der Rotierring 12 relativ zum Mechanikhalter 5 verdreht werden kann. Die Gewindestange weist an der in Figur 1 und 2 gezeigten oberen und der nicht gezeigten unteren Seite ein Gewindeprofil 8a auf und ist an der in Figur 1 gezeigten vorderen und der gegenüberliegenden Seite abgeflacht, so dass die Gewindestange 8 in einer in Figur 2 gezeigten Lagerung oder Durchgangsöffnung 12a des Rotierrings 12, welche dem Querschnitt der Gewindestange 8 in etwa entspricht, geführt werden kann, wodurch die Gewindestange 8 im Rotierring 12 verdrehsicher gelagert werden kann.

Der Mechanikhalter 5 weist in der gezeigten Ausführungsform vier Durchgänge 5a auf, durch welche die Einsteckelemente 4' des Löseteils 4 hindurchgeführt werden können, um das Löseteil 4 verdrehgesichert mit dem Mechanikhalter 5 zu verbinden bzw. in den Mechanikhalter 5 einstecken zu können.

An der den Einsteckelementen 4' des Löseteils 4 abgewandten Oberfläche des Löseteils 4 ist eine Rasterung 4a vorgesehen, welche in eine korrespondierende Rasterung 3a des Verschlussteiles 3 eingreifen kann, wenn das Verschlussteil 3 und das Löseteil 4 aufeinanderliegen, wodurch das Verschlussteil 3 und Lösteil 4 so gekoppelt sind, dass eine Drehung übertragen werden kann. Die zwischen der Gewindemutter 1 und dem Verschlussteil 3 angeordnete Druckfeder 2 bewirkt, dass im zusammengesetzten in Figur 3 gezeigten Zustand das Verschlussteil 3 und das Löseteil 4 immer aufeinander aufliegen.

Die Gewindemutter weist Nocken 1a auf, welche in Ausnehmungen 3b des Verschlussteiles 3 eingreifen und so eine Verdrehsicherung zwischen Gewindemutter 1 und Verschlussteil 3 herstellen. Im Inneren der Gewindemutter 1 ist ein Innengewinde 1b vorgesehen, welches in das Außengewinde 8a der Gewindestange 8 eingreifen kann, so dass beispielsweise über eine Drehbewegung der Gewindemutter 1 die verdrehsicher im Rotierring 12 gelagerte Gewindestange 8 in axialer Richtung der Vorrichtung, z.B. in der in Figur 1 gezeigten Darstellung nach links bewegt werden kann.

Das Verschlussteil 3 ist über Nocken 3' verdrehsicher in korrespondierenden Nuten 5b des Mechanikhalters 5 in der in Figur 3 gezeigten Ausgangsstellung gelagert.

Am hinteren in Figur 2 rechts gezeigten Ende der Gewindestange 8 ist eine als Rückstellelement dienende Schraubenfeder 7 angeordnet, welche im zusammengesetzten Zustand auf der einen Seite durch den Kopf der Gewindestange 8 und auf der anderen Seite durch einen inneren Anschlag der Gewindemutter 1 begrenzt ist.

Ein Mechanikverschluss 6 und eine Dosierknopfkappe 9 verschließen die Mechanik und können als Endanschläge dienen.

In der in Figur 3 gezeigten Ausgangsstellung sind das Löseteil 4 und das Verschlussteil 3 so weit in den Mechanikhalter 5 eingeschoben, dass die zur Verdrehsicherung vorgesehenen Nocken 3' in die korrespondierenden Nuten 5b des Mechanikhalters 5 eingreifen, so dass das Verschlussteil 3 relativ zum Mechanikhalter 5 nicht gedreht werden kann und die mit dem Verschlussteil 3 über die Nocken 1a, welche in die Ausnehmungen 3b des Verschlussteiles 3 eingreifen, gehaltene Gewindemutter 1 ebenfalls relativ zum Mechanikhalter 5 verdrehgesichert ist. Die Gewindestange 8 ist in der in Figur 3 gezeigten Ausgangsstellung in einer hinteren Position und kann relativ zum Mechanikhalter 5 gedreht werden, da die Schleifscheiben 10 und 11 unbelastet aufeinanderliegen. Somit ist die Gewindestange 8 so weit nach hinten geschoben, dass von der Schraubenfeder 7 keine nach hinten wirkende Kraft an die Gewindestange 8 angelegt wird, welche die Gewindestange 8 noch weiter nach hinten verschieben könnte.

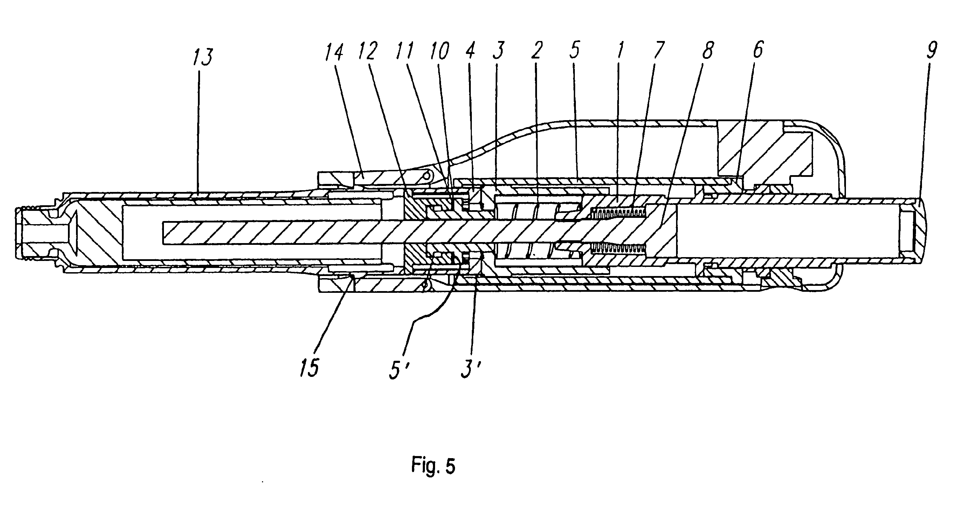

Wird eine Ampullenhülse 13 auf die in Figur 3 gezeigte Vorrichtung aufgesteckt, so schnappt diese Ampullenhülse 13 bei der Hinterschneidung 15 ein, wie in Figur 4 und 5 gezeigt. Der Rotierring 12 wird nach hinten bzw. in Figur 3 nach rechts geschoben, so dass die Schleifscheiben 10 und 11 aufeinanderliegen und den Rotierring 12 verdrehsicher mit dem Mechanikhalter 5 koppeln, wodurch auch die in der Durchgangsöffnung 12a gelagerte Gewindestange 8 verdrehgesichert wird. Des weiteren werden die Einsteckelemente 4' des Löseteils 4 durch die Durchgänge 5a des Mechanikhalters 5 hindurchgeschoben, wodurch das Löseteil 4 zusammen mit dem Verschlussteil 3 ebenfalls nach hinten geschoben wird. Das Verschlussteil 3 wird so weit nach hinten geschoben, dass die zur Verdrehsicherung mit dem Mechanikhalter 5 dienenden Nocken 3' aus den korrespondierenden Nuten 5b des Mechanikhalters 5 herausgeschoben werden, wodurch das Verschlussteil 3 relativ zum Mechanikhalter 5 gedreht werden kann. Hierdurch wird die mit dem Verschlussteil 3 gekoppelte Gewindemutter 1 relativ zum Mechanikhalter 5 freigegeben und kann gedreht werden.

Eine Drehung der Gewindemutter 1 führt über das Innengewinde 1b, welches mit dem Außengewinde 8a der durch den drehsicher gelagerten Rotierring 12 jetzt drehsicher gelagerten Gewindestange 8 gekoppelt ist, zu einer Axialbewegung der Gewindestange 8. Durch fortgesetzte Drehung der Gewindemutter 1 kann die Gewindestange 8 so weit in Richtung der Ampullenhülse 13 verschoben werden, bis die Gewindestange 8 wie in Figur 5 gezeigt in einer vorderen Position ist, in welcher das maximal aus der Ampulle zu verdrängende Volumen eingestellt ist. Die um die Gewindestange 8 herum angeordnete Schraubenfeder 7 ist durch das Verschieben der Gewindestange 8 maximal vorgespannt.

Ein Abgabevorgang wird durch einen Druck auf die Dosierknopfkappe 9 aufgelöst, wodurch die in Abhängigkeit von der gewünschten Dosis nach vorne ausgeschobene Gewindestange 8 auf einen Verdrängungskörper in der Ampulle 13 gedrückt wird, durch welchen die in der Ampulle gespeicherte Substanz verdrängt und aus einer Ampullenöffnung ausgegeben und z.B. injiziert wird.

Ist die Ampulle so weit entleert, dass diese ausgewechselt werden soll, so befindet sich die Gewindestange 8 in einer vorderen Position, wie in Figur 5 gezeigt. Wird die Ampullenhülse 13 mittels einer Druckvorrichtung 14 von der erfindungsgemäßen Vorrichtung gelöst, so schiebt die vordere Druckfeder 2 das Verschlussteil 3, das Löseteil 4 und den Rotierring 12 wieder nach vorne, wodurch das Verschlussteil 3 über die Nocken 3', welche in die entsprechenden Nuten 5b des Mechanikhalters 5 eingreifen, verdrehgesichert wird und somit die Gewindemutter 1 wieder verdrehgesichert ist.

Durch das Verschieben des Rotierrings 12 nach vorne wird die Schleifscheibe 11 von der Schleifscheibe 10 gelöst, d.h. diese Schleifscheiben 10, 11 liegen nicht mehr durch eine äußere Kraft aufeinander auf, wodurch der Rotierring 12 frei wird und nicht mehr verdrehgesichert ist, so dass die Gewindestange 8, welche im Rotierring 12 in der Lagerung 12a gelagert ist, ebenfalls gedreht werden kann. Die auf die Gewindestange 8 drückende Schraubenfeder 7 bewirkt, dass sich die Gewindestange 8 bis zu ihrer in Figur 3 gezeigten Ausgangsstellung in axialer Richtung der Vorrichtung zurückdreht. Somit ist ein manuelles Zurückdrehen der Gewindestange 8 durch einen Benutzer beim Wechseln einer Ampulle nicht mehr erforderlich, wodurch die Handhabung der erfindungsgemäßen Vorrichtung vereinfacht wird.

Anstelle der durch die Schleifscheiben 10 und 11 gebildeten Kupplung können auch andere Kupplungssysteme verwendet werden, wie z.B. eine durch mehrere Schleifscheiben gebildete Lamellenkupplung, eine durch kegelförmigen Hülsen gebildete Kegelschleifkupplung, eine z.B. durch ein gebogenes Federblech gebildete Sinuslamellenkupplung, oder eine durch Tellerfedern gebildete Kupplung.

## Patentansprüche

1. Vorrichtung zur dosierten Abgabe eines Produkts mit einem Gehäuseelement (5), einer Dosiervorrichtung (8), welche relativ zu dem Gehäuseelement (5) verschiebbar ist, einer Einstellvorrichtung (1), mit welcher die Position der Dosiervorrichtung (8) eingestellt werden kann und einem Rückstellelement (7), das durch eine Bewegung der Dosiervorrichtung (8) in eine Abgaberichtung eine Rückstellkraft erzeugt, durch welche die Dosiervorrichtung (8) in eine Ausgangsstellung zurückgebracht werden kann, eine erste Verdrehsicherung (10, 11, 12a), durch welche die Dosiervorrichtung (8) relativ zum Gehäuseelement (5) verdrehgesichert werden kann,
**gekennzeichnet durch**
eine zweite Verdrehsicherung (3, 4), **durch** welche die Einstellvorrichtung (1) relativ zum Gehäuseelement (5) verdrehgesichert werden kann,
wobei die erste Verdrehsicherung (10, 11, 12a) und die zweite Verdrehsicherung (3, 4) so gekoppelt sind, dass in einer ersten Position die Einstellvorrichtung (1) drehbar und die Dosiervorrichtung (8) verdrehgesichert ist und in einer zweiten Position die Einstellvorrichtung (1) verdrehgesichert und der Dosiervorrichtung (8) drehbar ist, so dass in der zweiten Position die Dosiervorrichtung (8) automatisch **durch** das Rückstellelement (7) in die Ausgangstellung zurückgebracht werden kann.

2. Vorrichtung nach Anspruch 1, wobei das Rückstellelement (7) ein Federelement ist.

3. Vorrichtung nach Anspruch 2, wobei das Federelement (7) eine Schraubenfeder oder eine Biegefeder ist.

4. Vorrichtung nach einem der vorhergehenden Ansprüche, wobei das Rückstellelement (7) um die Dosiervorrichtung (8) herum angeordnet ist.

5. Vorrichtung nach einem der vorhergehenden Ansprüche, wobei das Rückstellelement (7) im Wesentlichen koaxial zu dem Gehäuseelement (5), der Einstellvorrichtung (1) und/oder der Dosiervorrichtung (8) angeordnet ist.

6. Vorrichtung nach einem der vorhergehenden Ansprüche mit einer Sicherung (10, 11, 12), welche ein Rückstellen der Dosiervorrichtung (8) durch das Rückstellelement (7) verhindert.

7. Vorrichtung nach einem der vorhergehenden Ansprüche, wobei ein Gewindeeingriff zwischen der Einstellvorrichtung (1) und der Dosiervorrichtung (8) vorgesehen ist.

8. Vorrichtung nach einem der vorhergehenden Ansprüche, wobei die Einstellvorrichtung (1) ein Innengewinde und die Dosiervorrichtung (8) ein Außengewinde aufweist.

9. System mit einer Vorrichtung nach einem der vorhergehenden Ansprüche und einer Kopplungsvorrichtung (14, 15) für eine Ampullenhülse (13).

10. System nach dem vorhergehenden Anspruch, wobei durch die Kopplungsvorrichtung (14, 15) die Verdrehsicherung der Einstellvorrichtung (1) und/oder der Dosiervorrichtung (8) hergestellt oder gelöst werden kann.

## Claims

1. A device for dispensing a product in doses, comprising a casing element (5), a dosing device (8) which can be shifted relative to the casing element (5), a setting device (1) using which the position of the dosing device (8) can be set, and a restoring element (7) which uses a movement of the dosing device (8) in a dispensing direction to generate a restoring force by which the dosing device (8) can be returned to a starting position, and comprising a first rotational block (10, 11, 12a) using which the dosing device (8) can be secured against rotation relative to the casing element (5),
**characterised by**
a second rotational block (3, 4) using which the setting device (1) can be secured against rotation relative to the casing element (5),
wherein the first rotational block (10, 11, 12a) and the second rotational block (3, 4) are coupled such that in a first position, the setting device (1) can be rotated and the dosing device (8) is secured against rotation, and in a second position the setting device (1) is secured against rotation and the dosing device (8) can be rotated, such that in the second position, the dosing device (8) can be automatically returned to the starting position by the restoring element (7).

2. The device according to claim 1, wherein the restoring element (7) is a spring element.

3. The device according to claim 2, wherein the spring element (7) is a helical spring or a spiral spring.

4. The device according to any one of the preceding claims, wherein the restoring element (7) is arranged around the dosing device (8).

5. The device according to any one of the preceding claims, wherein the restoring element (7) is arranged substantially co-axially with the casing element (5), the setting device (1) and/or the dosing device (8).

6. The device according to any one of the preceding claims, comprising a securing mechanism (10, 11, 12) which prevents the dosing device (8) from being restored by the restoring element (7).

7. The device according to any one of the preceding claims, wherein a threaded engagement is provided between the setting device (1) and the dosing device (8).

8. The device according to any one of the preceding claims, wherein the setting device (1) comprises an inner thread and the dosing device (8) comprises an outer thread.

9. A system comprising a device according to any one of the preceding claims and a coupling device (14, 15) for an ampoule sleeve (13).

10. The system according to the preceding claim, wherein the rotational block for the setting device (1) and/or the dosing device (8) can be established or released by the coupling device (14, 15).

## Revendications

1. Dispositif pour la libération dosée d'un produit, avec un élément formant boîtier (5), un dispositif de dosage (8), déplaçable par rapport à l'élément formant boîtier (5), un dispositif de réglage (1), avec lequel la position du dispositif de dosage (8) peut être réglée, et un élément de rappel (7), produisant, au moyen d'un déplacement du dispositif de dosage (8) dans un sens de libération, une force de rappel au moyen de laquelle le dispositif de dosage (8) peut être replacé en une position initiale, une première sécurité de rotation (10, 11, 12a), au moyen de laquelle le dispositif de dosage (8) peut être assuré en rotation par rapport à l'élément formant boîtier (5),
**caractérisé**
**par** une deuxième sécurité de rotation (3, 4), au moyen de laquelle le dispositif de réglage (1) peut être assuré en rotation par rapport à l'élément formant boîtier (5),
où la première sécurité de rotation (10, 11, 12a) et la deuxième sécurité de rotation (3, 4) sont couplées de manière que, dans une première position, le dispositif de réglage (1) puisse tourner et le dispositif de dosage (8) soit assuré en rotation et, dans une deuxième position, le dispositif de réglage (1) soit assuré en rotation et le dispositif de dosage (8) puisse tourner, de manière que, dans le deuxième position, le dispositif de dosage (8) puisse être replacé automatiquement dans la position initiale au moyen de l'élément de rappel (7).

2. Dispositif selon la revendication 1, **caractérisé en ce que** l'élément de rappel (7) est un élément élastique.

3. Dispositif selon la revendication 2, **caractérisé en ce que** l'élément de rappel (7) est un ressort hélicoïdal ou un ressort de flexion.

4. Dispositif selon l'une des revendications précédentes, **caractérisé en ce que** l'élément de rappel (7) est disposé autour du dispositif de dosage (8).

5. Dispositif selon l'une des revendications précédentes, **caractérisé en ce que** l'élément de rappel (7) est disposé sensiblement coaxialement par rapport à l'élément formant boîtier (5), le dispositif de réglage (1) et/ou le dispositif de dosage (8).

6. Dispositif selon l'une des revendications précédentes, avec une sécurité (10, 11, 12) empêchant un rappel du dispositif de dosage (8) au moyen de l'élément de rappel (7).

7. Dispositif selon l'une des revendications précédentes, **caractérisé en ce qu'**une mise en prise par filetage est prévue entre le dispositif de réglage (1) et le dispositif de dosage (8).

8. Dispositif selon l'une des revendications précédentes, **caractérisé en ce que** le dispositif de réglage (1) présente un filetage intérieur et le dispositif de dosage (8) présente un filetage extérieur.

9. Système équipé d'un dispositif selon l'une des revendications précédentes et d'un dispositif de couplage (14, 15) pour une douille à ampoule (13).

10. Système selon la revendication précédente, dans lequel la sécurité en rotation du dispositif de réglage (1) et/ou du dispositif de dosage (8) peut être établie ou annulée au moyen du dispositif de couplage (14, 15).
